Europäisches Patentamt

European Patent Office

Office européen des brevets

(11) Veröffentlichungsnummer: **0 451 427 A1**

(12) **EUROPÄISCHE PATENTANMELDUNG**

(21) Anmeldenummer: 90810343.5

(22) Anmeldetag: 07.05.90

(51) Int. Cl.5: **A61F 2/36**, A61F 2/46, A61B 17/16

(30) Priorität: **10.04.90 CH 1211/90**

(43) Veröffentlichungstag der Anmeldung: **16.10.91 Patentblatt 91/42**

(84) Benannte Vertragsstaaten: **AT BE DE ES FR GB IT NL SE**

(71) Anmelder: **GEBRÜDER SULZER AKTIENGESELLSCHAFT Zürcherstrasse 9 CH-8401 Winterthur(CH)**

(72) Erfinder: **Zweymüller, Karl, Prof.Dr-med. Orthopädische Univ.-Klinik, Garnisonstrasse 13 A-1090 Wien(AT)**
Erfinder: **Koch, Rudolf Oberdorfstrasse 229 CH-8267 Berlingen(CH)**

(54) Verankerungsschaft für eine Femurkopfprothese.

(57) Um eine Primärfixierung einer zuvor als Deckel vom Femurknochen (16) abgehobenen Knochenschale (15) zu erreichen, ist der Prothesenschaft (1) im proximalen Bereich mit einem Längsschlitz (5) parallel zur seiner Längsachse versehen, an den eine Gleitbahn (9) anschliesst; in dieser ist ein Gleitstein (11) eines Gewindebolzens (10) verschiebbar gelagert, auf den eine hülsenartige Befestigungsmutter (14) aufgeschraubt ist. Durch deren Kopf wird die Knochenschale (15) am Gewindebolzen (10) und damit am Schaft (1) solange fixiert, bis sie mit dem Femur (16) wieder zusammengewachsen ist.

Fig. 1

Die Erfindung betrifft einen Verankerungsschaft für eine Femurkopfprothese, der mindestens im proximalen Bereich mindestens in einer seiner nach anterior oder posterior gerichteten Schaftoberflächen in Richtung seiner Längsachse mit einem Schlitz für den Einschub eines Bauteils versehen ist.

Eine Prothese der genannten Art ist beispielsweise aus der EP-B-0 217 034 bekannt.

Für Reoperationen ist in neuerer Zeit eine Operationstechnik entwickelt worden, bei der ein Teil der die zu reoperierende Prothese umgebenden kortikalen Knochenschale vollständig vom übrigen Knochen gelöst und als im wesentlichen in Knochenlängsrichtung verlaufender Deckel abgehoben wird. Ist die neue Prothese eingesetzt, so wird der Knochendeckel wieder aufgesetzt. Bei dieser Art von Reoperationen besteht das Problem, den Knochendeckel primär zu fixieren, bis er wieder mit dem restlichen Knochen verwachsen ist. Aufgabe der vorliegenden Erfindung ist es, dieses Problem zu lösen.

Die Lösung erfolgt nach der vorliegenden Erfindung dadurch, dass der Schlitz eine Gleitbahn aufweist, in der ein Gleitstein mindestens eines in dem Schlitz verschiebbaren Gewindebolzens gelagert ist.

Im Berich des Schlitzes, dessen Achse zweckmässigerweise parallel zur zugehörigen Oberfläche verläuft, lassen sich ein oder vorteilhafterweise meistens mindestens zwei Gewindebolzen in einer beliebigen Höhe im proximalen Bereich des Knochens verschieben. Durch Bohrungen in der erwähnten Knochenschale hindurch werden dann Befestigungsmuttern für diese Schale auf den Bolzen fixiert.

Bei einer bevorzugten Ausführungsform der Erfindung besteht die Gleitbahn in einer Längsbohrung am proximalen Schaftende, während der Gleitstein einen zylinder- oder kugelförmigen Führungskörper aufweist, dessen Durchmesser an den Durchmesser der Gleitbahnbohrung angepasst ist. Die Befestigungsmuttern können mit Vorteil durch eine Hülse mit einem pilzförmigen Kopf realisiert sein, wobei für die Montage der Hülsenkopf mit einem Innensechskant und/oder mit zu seiner Achse parallelen, über den Umfang verteilten Sacklöchern versehen sein kann. Für das Anbringen eines Sicherungselementes, beispielsweise eines Drahtes, gegen ein unbeabsichtiges Lösen der Schraubverbindung zwischen Hülsenkopf und Gewindebolzen kann man den Hülsenkopf darüberhinaus mit Bohrungen senkrecht zu seiner Achse versehen.

Um die Montage des Gewindebolzens in dem Schlitz zu erleichtern, kann man den Schlitz bis zum proximalen Ende des Schaftes erstrecken. Eine Verwendung der neuen Prothese in beiden Oberschenkeln wird ermöglicht, wenn beide nach anterior oder posterior weisenden Schaftoberflächen mit Schlitzen versehen sind.

Die Montage der Befestigungsmuttern wird erleichtert, wenn der Gewindebolzen mit einer Bohrung, z.B. mit einem Innensechskant, versehen ist.

Für das Anbringen der Bohrungen für die Aufnahme der Muttern oder Hülsen in der kortikalen Schale hat sich ein Instrument bewährt mit einem in Form und Länge an die Gleitbahn des Schlitzes angepassten Stamm, der einen im Schlitz geführten Nocken aufweist, ferner mit einer lösbar mit dem Stamm verbundenen rechtwinkligen Schiene, deren einer Schenkel parallel zu Gleitbahn und Schlitz verläuft, und schliesslich mit einer Längsausnehmung in den zum Schlitz parallelen Schenkeln, deren Länge der Schlitzlänge entspricht und in der mindestens eine Bohrlehre verschiebbar und fixierbar gelagert ist.

Für eine Einstellmöglichkeit auf individuelle Knochendicken der Patienten kann in dem anderen Schenkel des Instrumentes ein Langloch vorgesehen sein.

Im folgenden wird die Erfindung anhand eines Ausführungsbeispiels im Zusammenhang mit der Zeichnung näher erläutert.

| | |
|---|---|
| Fig. 1 | zeigt, teilweise im Schnitt, eine Ansicht des proximalen Bereiches des neuen Schaftes; |
| Fig. 2 | ist eine Ansicht von Fig. 1 von links; |
| Fig. 3 | ist der Schnitt III-III von Fig. 2; die |
| Fig. 4 - 6 | geben verschiedene Ausführungsformen des Gewindebolzens wieder; |
| Fig. 7 | zeigt in einem Ausschnitt aus Fig. 1 das Instrument für das Setzen der Bohrungen in der Knochenschale; |
| Fig. 8 | ist eine Ansicht von Fig. 7 von links, während |
| Fig. 9 und 10 | je eine Aufsicht auf Fig. 7 wiedergeben. |

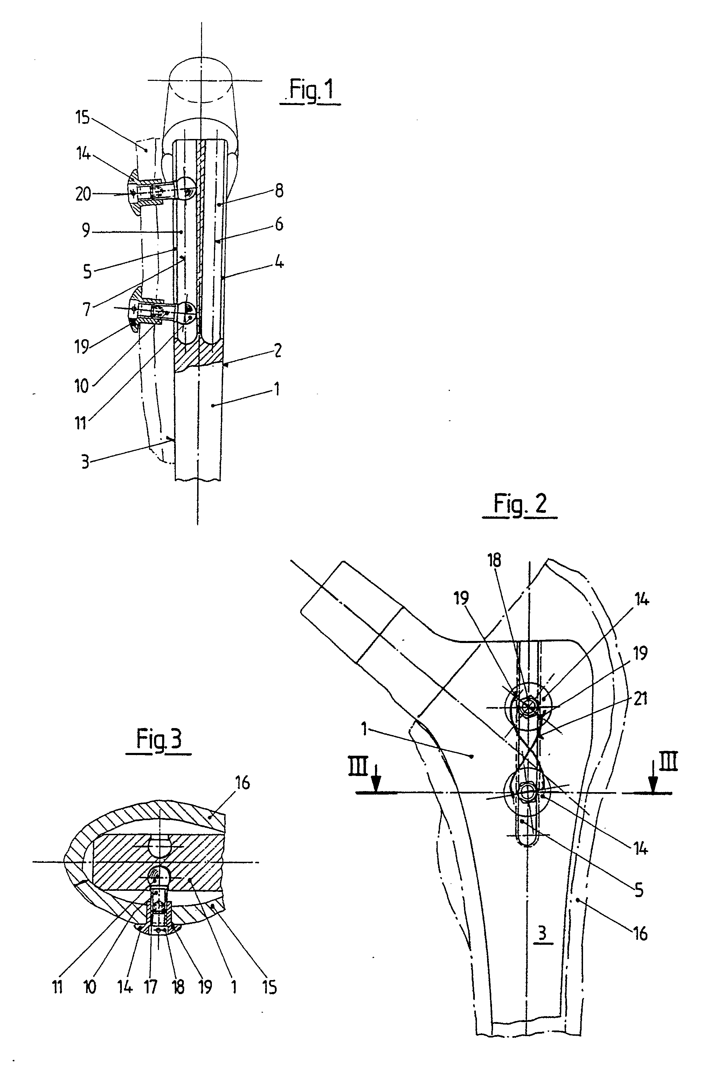

Der als Beispiel gewählte Schaft 1 (Fig. 1) ist als sogenannter Geradschaft mit nach anterior bzw. posterior weisenden, ebenen Blattseiten 2 und 3 ausgebildet. Er erweitert sich von seinem nicht dargestellten distalen Ende nach proximal allseitig stetig. In seinem proximalen Bereich ist in jeder Blattseite 2, 3 ein Schlitz 4, 5 vorgesehen, dessen Achse 6, 7 parallel zur Oberfläche der zugehörigen Blattseite 2 bzw. 3 verläuft. Die Schlitze 4, 5 stehen mit Gleitbahnen 8, 9 in Verbindung, die als Längsbohrungen vom proximalen Ende in den Schaft 1 eingebracht sind. Schlitze 4, 5 und Gleitbahnen 8, 9 sind daher oben offen, was das Einsetzen der Gewindebolzen 10 mit ihren Gleitsteinen 11 erleichtert.

Die Gleitsteine 11 sind in ihren Abmessungen an den Bohrungsdurchmesser der Gleitbahn 8, 9 angepasst und können beispielsweise als Zylinder (Fig. 4), Kugel (Fig. 5) oder "Fass" (Fig. 6) ausgebildet sein, wobei alle Ausführungsformen in gewissem Umfang ein seitliches Schwenken der Achse der Gewindebolzen 10 ermöglichen, die Kugelform darüberhinaus jedoch ein zusätzliches Verschwenken dieser Achsen nach oben oder unten erlaubt.

Senkrecht zur Achse des Gleitsteines 11 ist auf seinem Umfang ein Gewindebolzen 10 angesetzt, der ein Gewinde 12 trägt und mit einem Innensechskant 13 für den Einsatz eines nicht gezeigten Haltewerkzeuges beim Aufschrauben einer Befestigungsmutter 14 versehen ist, durch die die kortikale Knochenschale 15 (Fig. 3) nach der Implantation der Prothese gemäss dem eingangs beschriebenen Vorgehen auf dem Femur 16 (Fig. 2 und 3) fixiert wird, bis ein Zuammenwachsen beider Knochenteile 15, 16 erfolgt ist.

Die Befestigungsmutter 14 besteht im vorliegenden Beispiels aus einer Hülse mit einem Innengewinde 17 und einem pilzförmigen Kopf, in den ein weiterer Innensechskant 18 für ein Werkzeug zum Aufschrauben der Hülse 14 auf den Bolzen 10 vorgesehen ist. Für den Ansatz eines Werkzeuges können jedoch im Kopf der Hülse 14 auch kleine Sackbohrungen 19 vorgesehen sein, die über den Umfang des Kopfes verteilt sind.

Weiterhin sind im Hülsenkopf kleine Bohrungen 20 vorhanden, die senkrecht zu seiner Achse verlaufen und zum Durchziehen eines Drahtes 21 dienen, der, wie in Fig. 2 gezeigt, ein Sicherungselement gegen ein unbeabsichtigtes Lösen der Verschraubung 11, 17 darstellt.

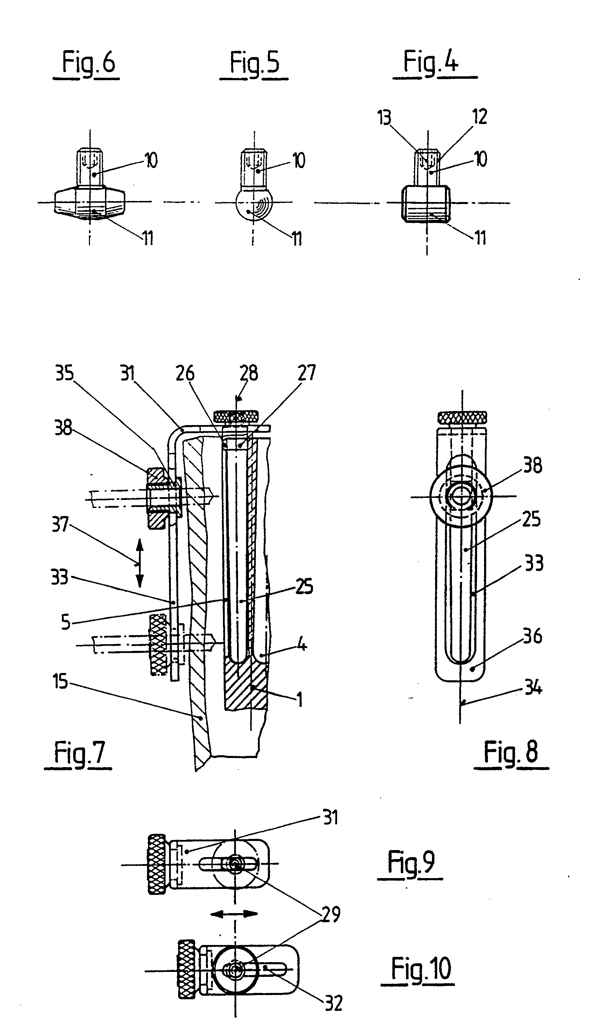

Eine Grundvoraussetzung für die Wirksamkeit der neuen Prothese besteht darin, dass die Durchtrittsbohrungen durch die Knochenschale 15 für die Hülse 14 sich am richtigen Ort befinden und in der richtigen Richtung verlaufen. Um ihr "Setzen" zu erleichtern ist ein Instrument (Fig. 7) vorgesehen, das einen Stamm 25 aufweist, der nach distal leicht konisch geformt ist und in seiner Länge an die Länge der Gleitbahn 9 angepasst ist. Der Stamm 25 ist oben als Zylinder 27 ausgebildet, der auf den Durchmesser der Gleitbahn 9 abgestimmt ist; zusätzlich ist an den Zylinder 27 ein Hocken 26 angesetzt, der in den Schlitz 5 eingreift. Zylinder 27 und Hocken 26 zentrieren und halten den Stamm 25 in der Gleitbahn 9 derart, dass seine Achse 28 mit der Achse 7 zusammenfällt. Stirnseitig ragt aus dem Stamm 25 ein Gewindebolzen 29 (Fig. 9) heraus, auf den eine Rändelmutter als Klemmschraube 30 aufgeschraubt werden kann.

Mit dieser Schraube 30 wird eine rechtwinklige Schiene 31 auf dem Stamm 25 befestigt, die für den Durchtritt des Bolzens 29 ein Langloch 32 aufweist, in dem die Schiene 31 in Richtung des Doppelpfeiles zwischen Fig. 9 und 10 verschoben werden kann, um die Abstände zwischen der Achse 28, 7 und der äusseren Oberfläche der Knochenschale 15 einstellen zu können, um so unterschiedlichen Knochendicken Rechnung zu tragen.

Der vertikale zweite Schnekel 36 der Schiene 31, dessen Achse 34 parallel zur Gleitbahnachse 7 und zum Schlitz 5 verläuft, hat eine Längsausnehmung 33, in der eine Bohrlehre 35 verschiebbar (Fig. 7: Doppelpfeil 37) angeordnet ist und mit Hilfe einer Rändelmutter 38 in beliebiger Höhe fixiert werden kann. Sobald die Bohrlehre 35 am richtigen Ort für die Durchtrittsbohrung in der Knochenschale 15 fixiert isr, werden Bohrungen 39 in die Knochenschale 15 gebohrt.

**Patentansprüche**

1. Verankerungsschaft (1) für eine Femurkopfprothese, der mindestens im proximalen Bereich mindestens in einer seiner nach anterior oder posterior gerichteten Schaftoberflächen (2, 3) mit einem Schlitz (4,5) in Richtung seiner Längsachse für den Einschub eines Bauteils versehen ist, dadurch gekennzeichnet, dass der Schlitz (4, 5) eine Gleitbahn (8, 9) aufweist, in der ein Gleitstein (11) mindestens eines in dem Schlitz (4, 5) verschiebbaren Gewindebolzens (10) gelagert ist.

2. Schaft nach Anspruch 1, dadurch gekennzeichnet, dass der Schlitz (4, 5) sich bis zum proximalen Ende des Schaftes (1) erstreckt.

3. Schaft nach Anspruch 1 oder 2, dadurch gekennzeichnet, dass die Achse (6,7) des Schlitzes (4,5) parallel zu der zugehörigen Schaftoberfläche (2,3) verläuft.

4. Schaft nach einem der Ansprüche 1 bis 3, dadurch gekennzeichnet, dass die Gleitbahn (8,9) in einer Längsbohrung am proximalen Schaftende besteht.

5. Schaft nach Anspruch 4, dadurch gekennzeichnet, dass der Gleitstein (11) einen zylinder- oder kugelförmigen Führungskörper aufweist, dessen Durchmesser an den Durchmesser der Gleitbahnbohrung angepasst ist.

6. Schaft nach einem der Ansprüche 1 bis 5, dadurch gekennzeichnet, dass der Gewindebolzen (10) mit einem Innensechskant (13) versehen ist.

7. Schaft nach Anspruch 1 bis 6, dadurch gekennzeichnet, dass auf den Gewindebolzen (10) eine Hülse (14) mit einem pilzförmigen Kopf aufgeschraubt ist.

8. Schaft nach Anspruch 7, dadurch gekennzeichnet, dass der Hülsenkopf ebenfalls mit einem Innensechskant (18) versehen ist.

9. Schaft nach Anspruch 7, dadurch gekennzeichnet, dass der Hülsenkopf mit zu seiner Achse parallelen, über den Umfang verteilten Sacklöchern (19) versehen ist.

10. Schaft nach einem der Ansprüche 7 bis 9, dadurch gekennzeichnet, dass in dem Hülsenkopf Bohrungen (20) senkrecht zu seiner Achse vorgesehen sind.

11. Schaft nach einem der Ansprüche 1 bis 11 dadurch gekennzeichnet, dass in der anderen Oberfläche (2) ein gleichartiger Schlitz (4) vorgesehen ist.

12. Schaft nach einem der Ansprüche 1 bis 12, dadurch gekennzeichnet, dass in einem Schlitz (4, 5) mindestens zwei Gewindebolzen (10) mit aufgeschraubter Hülse (14) vorhanden sind.

13. Instrument zum Festlegen der Lage der Bohrungen in der kortikalen Knochenschale (15), gekennzeichnet durch einen in Form und Länge an die Gleitbahn (8, 9) des Schlitzes (4, 5) angepassten Stamm (25), der einen im Schlitz (4, 5) geführten Nocken (26) aufweist, ferner durch eine lösbar mit dem Stamm (25) verbundene, rechtwinklige Schiene (31), deren einer Schenkel (36) parallel zu Gleitbahn (8, 9) und Schlitz (4, 5) verläuft, und schliesslich durch eine Längsausnehmung (33) in dem zum Schlitz (4, 5) parallelen Schenkel (36), deren Länge der Schlitzlänge entspricht, und in der eine Bohrlehre (35) verschiebbar und fixierbar gelagert ist.

14. Instrument nach Anspruch 14, gekennzeichnet durch ein Langloch (32) in dem anderen Schenkel der Schiene (31).

Fig. 1

Fig. 2

Fig. 3

Fig.6  Fig.5  Fig.4

Fig.7  Fig.8

Fig.9

Fig.10

**Europäisches
Patentamt**

# EUROPÄISCHER
# RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-3 939 498　(LEE)<br>* Spalte 2, Zeilen 11-36; Figuren *<br>– – – | 1-5 | A 61 F 2/36<br>A 61 F 2/46<br>A 61 B 17/16 |
| D,Y | EP-A-0 217 034　(SULZER)<br>* Ganzes Dokument *<br>– – – | 1-5 | |
| A | GB-A-1 442 990　(ENGLISH)<br>– – – | | |
| A | US-A-4 913 137　(AZER)<br>* Zusammenfassung; Abbildung 15; Spalte 7, Zeile 51 - Spalte 8, Zeile 39 *<br>– – – | 13 | |
| A | US-A-3 670 724　(BOSACCO)<br>– – – – – | | |

| RECHERCHIERTE SACHGEBIETE (Int. Cl.5) |
|---|
| A 61 F<br>A 61 B |

Der vorliegende Recherchenbericht wurde für alle Patentansprüche erstellt

| Recherchenort | Abschlußdatum der Recherche | Prüfer |
|---|---|---|
| Den Haag | 10 Dezember 90 | STEENBAKKER J. |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder
nach dem Anmeldedatum veröffentlicht worden ist
D : in der Anmeldung angeführtes Dokument
L : aus anderen Gründen angeführtes Dokument

-----------------------------------------------------------------------------------

& : Mitglied der gleichen Patentfamilie,
übereinstimmendes Dokument